# EUROPÄISCHE PATENTANMELDUNG

(11) **EP 2 353 539 A1**
(43) Veröffentlichungstag der Anmeldung: **10.08.2011**
(21) Anmeldenummer: 11000469.4
(22) Anmeldetag: 21.01.2011
(51) Int. Cl.: A61C 1/00, B25F 3/00

(54) **Arbeitsgerät mit wechselbarem Arbeitskopf**

(30) Priorität: 01.02.2010 DE 102010006549
(71) Anmelder: Dürr Dental AG, 74321 Bietigheim-Bissingen (DE)
(72) Erfinder: Hatzfeld, Falk, 74379 Ingersheim (DE)
(74) Vertreter: Ostertag, Reinhard

(57) **Zusammenfassung**

Für die Identifizierung von dentalen Handstücken (10), die über ein Basisteil (18) mit einer Versorgungseinheit (12) verbunden sind, wird vorgeschlagen, einen beim Schließen der Verbindungen zwischen Handstück (10) und Versorgungseinheit (12) einsetzenden Versorgungsstrom auf seine Größe auszuwerten. Dabei wird, gegebenenfalls durch Vorsehen von Identifizierungswiderständen im Handstück (10) gewährleistet, dass der nach dem Verbinden von Handstück (10) und Versorgungseinheit (12) fließende Versorgungsstrom eindeutig vom jeweils angesetzten Handstück (10) abhängt. Durch einen Strommesser (30, 32) der Versorgungseinheit und einen nachgeschalteten Stromdiskriminator (36, 42) kann dann ein Signal erzeugt werden, welches das angesetzte Handstück (10) eindeutig identifiziert.

## Beschreibung

Die Erfindung betrifft ein Arbeitsgerät mit einem wechselbaren Arbeitskopf, insbesondere einem dentalen Handstück.

Derartige Arbeitsgeräte sind z.B. in Form von dentalen Bohrgeräten bekannt, welche auswechselbare Bohrköpfe haben, die sich insbesondere in der Arbeitsdrehzahl und in dem Winkel unterscheiden, unter welchem der Bohrer zur Werkstückachse geneigt ist.

Für viele Anwendungsfälle wäre es nun wünschenswert, wenn man bei einem Arbeitsgerät mit wechselbarem Arbeitskopf auf einfache Weise unabhängig von einer visuellen Kontrolle feststellen könnte, welcher Arbeitskopf sich gerade am Versorgungsadapter (oder Basisteil) befindet, über welchen der Arbeitskopf von einer Versorgungseinheit her mit Energie oder Verbrauchsmitteln wie Luft und Wasser versorgt wird.

Zur Lösung dieser Aufgabe schafft die Erfindung ein Arbeitsgerät mit den im Anspruch 1 angegebenen Merkmalen.

Man kann zwar im Prinzip auswechselbare Geräteteile mit maschinell lesbaren Marken versehen, die dann ausgelesen werden können, um zu kontrollieren, ob der richtige Arbeitskopf angebracht ist. Derartige Marken wie Barcode-Etiketten oder RFID-Marken und die zugehörigen Leseköpfe benötigen aber viel Platz. Viele Arbeitsköpfe, wie z.B. dentale Handstücke, müssen aber aus ergonomischen Gründen und/oder im Hinblick auf den Einsatzort sehr schlank gewählt werden, so dass für optische Marken oder RFID-Marken kein Raum bleibt.

Bei dem erfindungsgemäßen Arbeitsgerät wird davon ausgegangen, dass für den dem Arbeitskopf zugeführten Versorgungsstrom ein für den Arbeitskopf typischer Widerstand angetroffen wird. Durch Messen dieses Widerstandes über den Versorgungsstrom erhält man somit eine Information, welche die Art des aufgesetzten Arbeitskopfes erkennen lässt.

Enthält ein Arbeitskopf keinen derartigen Verbraucher oder gibt es unterschiedliche Arbeitsköpfe mit gleichem Widerstand für den Versorgungsstrom, wird in den Arbeitskopf ein zusätzlicher charakteristischer Verbrauch eingefügt, der nur Identifizierungszwecken dient, so dass auch bei Arbeitsköpfen mit bezüglich des Verbrauches identischen Nutz-Verbrauchern eine Identifizierung des Arbeitskopfes möglich ist. Nutz-Verbraucher und Identifizierungs-Verbraucher sollen nachstehend zusammen unter Verbraucher angesprochen werden.

Die Widerstandsmessung erfolgt unter Verwendung eines Strommessers der Vesorgungseinheit. Da die Anzahl unterschiedlicher an einem Basisteil lösbar anbringbarer Arbeitsköpfe übersichtlich ist, reicht es aus, wenn man den Versorgungsstrom nur gob misst. Eine hohe Auflösung aufweisender Strommesser ist nicht notwendig.

Bei der Abfassung der Ansprüche und der nachstehenden Beschreibung wurde davon ausgegangen, dass die treibende Kraft des Versorgungsstromes (Spannung bei elektrischem Strom, Druck bei Fluidstrom) konstant gehalten wird. Es versteht sich, dass man dann, wenn dies nicht gewährleistet werden kann, sowohl die treibende Kraft als auch den Strom messen muss und hieraus dann den Widerstand bestimmen muss, der letztlich die entscheidende Größe ist. Der besseren Lesbarkeit halber wird in den Ansprüchen und in der Beschreibung aber auf eine Strommessung abgehoben.

Der Versorgungsstrom wird in der Mehrzahl der Fälle ein elektrischer Strom sein. Das erfindungsgemäße Prinzip zur Erkennung von Arbeitsköpfen lässt sich aber auch auf Fluidströme übertragen, wie für den Fachmann leicht nachvollziehbar ist.

In den Ansprüchen und der Beschreibung wird von einer Strommessung gesprochen. Diese Messung kann die Amplitude (ohmsche Verbraucher) und/oder die Phasenlage des Stromes (Verbraucher mit Scheinwiderstand) umfassen. Beides soll unter dem Wort Strommessung gemeinsam verstanden werden.

Vorteilhafte Weiterbildungen der Erfindung sind in Unteransprüchen angegeben.

Ein Arbeitsgerät gemäß Anspruch 2 kann unterschiedliche Arbeitsköpfe aus einem ganzen Satz mit dem Arbeitsgerät verwendbarer Arbeitsköpfe erkennen.

Bei einem Arbeitsgerät gemäß Anspruch 3 wird die Auswertung des Versorgungsstromes im Hinblick auf ein erkennen des Arbeitskopfes nach einer vorgegebenen Zeit beendet. Man kann dann den Versorgungsstrom im Hinblick auf andere Kriterien überwachen, z.B. Über-Schreitung eines maximal zulässigen Arbeitsstromes oder dergleichen.

Bei einem Arbeitsgerät gemäß Anspruch 4 wird automatisch angezeigt, wenn sich der eingesetzte Arbeitskopf von einem an sich zu verwendenden Arbeitskopf unterscheidet. Bei regelmäßig vorgenommenen Routinearbeiten kann die Sollidentifizierungssignalquelle einfach ein Datenträger sein, auf dem die nacheinander zu verwendenden Arbeitsköpfe aufgezeichnet sind.

Bei Arbeiten, die variabel durchgeführt werden müssen, kann man gemäß Anspruch 5 als Sollidentifizierungssignalquelle eine gesprochene Bezeichnung für den Arbeitskopf wählen. Ein Arzt kann z.B. mündlich von einer Helferin einen bestimmten Arbeitskopf anfordern, und beim Einsetzen dieses Arbeitkopfes in das Arbeitsgerät wird dann automatisch geprüft, ob dieser dem angeforderten Kopf entspricht oder nicht.

Bei einem Arbeitsgerät gemäß Anspruch 6 wird die Identifizierung des eingesetzten Arbeitskopfes nach einer vorgegebenen zur Identifizierung ausreichenden Zeitspanne beendet, und ab diesem Zeitpunkt kann dann der Versorgungsstrom im Hinblick auf andere Kriterien überwacht werden, z.B. Erkennen von Überlast oder plötzlicher Reduzierung der Last. Letzteres kann z.B. andeuten, dass ein Bohrer beginnt, in einen Hohlraum einzudringen, der aber nicht eröffnet werden soll.

Bei einem Arbeitsgerät gemäß Anspruch 7 ist es möglich, einen oder mehrere Arbeitsparameter des Arbeitskopfes durch die Versorgungseinheit mit überwachen zu lassen und einen Alarm zu erzeugen oder Gegenmaßnahmen zu ergreifen, wenn ein Arbeitsparameter einen zulässigen Parameterbereich verlässt.

Bei einem Arbeitsgerät gemäß Anspruch 8 ist es möglich, von der Versorgungseinheit her Informationen und Befehle an den Arbeitskopf zu übermitteln.

Sowohl die Signalübertragung vom Arbeitskopf zur Versorgungseinheit als auch die Signalübertragung von der Versorgungseinheit zum Arbeitskopf benötigt dabei keine zusätzlichen Verbindungsleiter zwischen Arbeitskopf und Versorgungseinheit.

Mit der Weiterbildung der Erfindung gemäß Anspruch 9 wird erreicht, dass die von der Versorgungseinheit zum Arbeitskopf gesandten Befehlsignale sehr einfach vom dem vom Arbeitskopf zur Versorgungseinheit übermittelten Arbeitsparametersignalen unterschieden werden können und voneinander getrennt werden können.

Bei einem Arbeitsgerät gemäß Anspruch 10 ist gewährleistet, dass seine Erkennung auch dann gewährleistet ist, wenn ein Nutzverbraucher des Arbeitskopfes noch nicht eingeschaltet ist.

Die Weiterbildung der Erfindung gemäß Anspruch 11 ist im Hinblick auf eine Reduzierung der vorzusehenden Leiter zwischen Arbeitskopf und Versorgungseinheit von Vorteil. Das Kleinhalten dieser Anzahl ist deshalb wichtig, weil die hier in Betracht gezogenen Arbeitsgeräte oft sehr schlank bauen müssen und neben elektrischen Leitern oft auch Fluidkanäle, insbesondere Luftkanäle oder Saugkanäle umfassen müssen, die großen Querschnitt haben müssen.

Wenn ein Arbeitsgerät einen Identifizierungsverbraucher enthält, kann man mit der Weiterbildung der Erfindung gemäß Anspruch 12 sicherstellen, dass der Identifizierungsverbraucher nach dem Erkennen des Arbeitskopfes keine Leistung mehr verbraucht.

Nachstehend wird die Erfindung anhand von Ausführungsbeispielen unter Bezugnahme auf die Zeichnung näher erläutert. In dieser zeigen:
- Figur 1: ein dentales Arbeitsgerät mit einem dentalen Handstück und einer dazugehörigen Versorgungseinheit;
- Figuren 2 bis 4: schematische Blockschaltbilder der Verbraucher unterschiedlicher Typen von Arbeitsköpfen;
- Figur 5: eine ähnliche Ansicht wie Figur 1, wobei ein Arbeitsgerät gezeigt ist, bei welchem Daten zwischen Arbeitskopf und Versorgungseinheit in beiden Richtungen ausgetauscht werden können; und
- Figur 6: ein Blockschaltbild eines Ultraschall-Handstückes mit zugehöriger Versorgungseinheit.

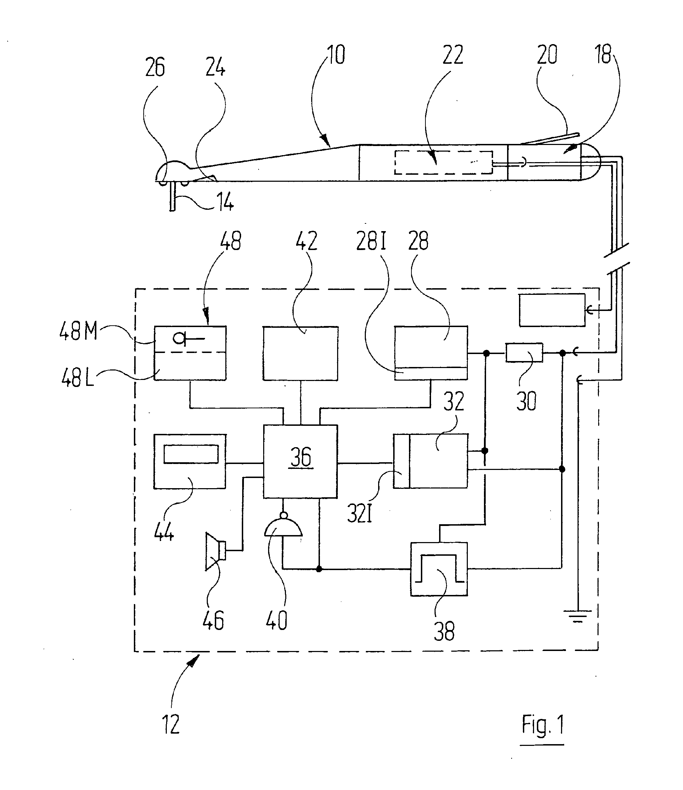

In Figur 1 ist mit 10 insgesamt ein dentales Handstück bezeichnet, mit 12 die zugeordnete Versorgungseinheit.

Vom Handstück 10 ist angenommen, dass es sich um ein Bohrer-Handstück handelt. Ein Bohrer ist bei 14 gezeigt. Die Achse des Bohrers steht senkrecht auf der Achse des Handstückes 10.

Das Handstück 10 ist lösbar auf ein Basisteil 16 aufgesetzt, welches über einen Versorgungsschlauch 18 mit der Versorgungseinheit 12 verbunden ist.

Ein Hebel 20 dient dazu, bei Bedarf das Handstück 10 vom Basisteil 16 durch Lösen einer mechanischen Verriegelung (nicht gezeigt) freizugeben.

Im Handstück 10 ist global ein Verbraucher 22 gezeigt, der einen oder mehrere elektrische Verbraucher und einen oder mehrere Fluidverbraucher umfassen kann. Typisch für ein Bohrer-Handstück 10 sind ein Antriebsmotor für den Bohrer 14, eine Lichtquelle 24 zum Beleuchten der Arbeitsstelle und Düsen 26 zu nennen, aus denen Wasser abgegeben wird.

Für die nachstehende Beschreibung werden der Übersichtlichkeit halber zunächst nur die elektrischen Verbraucher näher berücksichtigt.

Die Versorgungseinheit 12 umfasst ein Netzteil 28, welches den Verbraucher 22 mit elektrischer Energie versorgt. Der Strom, der zum Handstück 10 fließt und von dort wieder gegen Messe zurückfließt, wird durch einen Widerstand 30 gemessen. Die Klemmen des Widerstandes 30 sind mit einem Spannungsmesser 32 verbunden, welcher eine Digitalisierungsstufe 34 enthält.

Das so digitalisierte Stromsignal wird auf einen Eingang eines Prozessrechners 36 gegeben.

Die am Widerstand 30 abfallende Spannung wird ferner auf die Steuerklemme einer monostabilen Kippstufe 38 gegeben, die durch eine ansteigende Flanke des Stromsignales getriggert wird. Das Ausgangssignal der monostabilen Kippstufe 38 zeigt dem Prozessrechner 36 an, dass nun eine Arbeitsphase folgt, in welcher eine Identifizierung des Handstückes 10 anhand des Ausgangssignales des Strommessers 32 erfolgen soll.

Durch einen Inverter 40 erhält der Prozessrechner 36 auch das invertierte Ausgangssignal der Kippstufe 38, und dieses zeigt an, dass die Identifizierung des Handstückes 10 beendet ist und das Ausgangssignal des digitalen Spannungsmessers 32 nun für andere Zwecke verwendet werden kann, z.B. zum Erkennen von Überlastbedingungen.

Der Prozessrechner 36 arbeitet mit einem Speicher 42 zusammen, in welchem u. a. eine Tabelle gelegt ist, die drei Spalten enthält: In einer ersten Spalte stehen bestimmte Strombereiche, die jeweils für ein Handstück 10 charakteristisch sind. In einer zweiten Spalte steht ein Code für das entsprechende Handstück, und in einer dritten Spalte stehen z.B. Strombereiche, die im normalen Betrieb des Handstückes 10 eingehalten werden sollen.

Dadurch, dass der Prozessrechner 36 den beim Einstecken des Handstückes 10 in das Basisteil 16 liegenden Strom misst, kann er unter Verwendung der im Speicher 42 abgelegten Tabelle erkennen, welches Handstück 10 auf das Basisteil 16 aufgesetzt wurde. Das Ergebnis dieser Identifizierung zeigt der Prozessrechner 36 auf einem Display 44 an. Zusätzlich kann eine entsprechende Sprachausgabe auf einem Lautsprecher 46 erfolgen.

Bei dem soweit beschriebenen Arbeitsgerät muss der Benutzer selbst wissen, welches Handstück 10 er einsetzen wollte, und er muss dann die Rückmeldung durch Display 44 und Lautsprecher 46 mit seinem Handstückwunsch vergleichen.

In der Praxis ist es oft so, dass bei Operationen und anderen Arbeiten mit Bohrgeräten die jeweils benötigten Arbeitsgeräte oder Handstücke auf mündliche Anforderung an den Zahnarzt gegeben werden.

In der Versorgungseinheit 12 ist eine Befehlssignalquelle 48 enthalten, die ein Mikrofon 48M und eine Spracherkennungslogik 48L umfasst.

Der das Handstück verwendende Arzt kann somit ein gewünschtes Handstück mit einer Code-Bezeichnung von der Helferin anfordern, die auch als Identifizierungs-String im Speicher 42 hinterlegt ist. Damit kann der Prozessrechner 36 nach Erkennen des Handstücks 10 nachprüfen, ob das mündlich angeforderte Handstück mit dem tatsächlich eingesetzten Handstück übereinstimmt.

Ist dies nicht der Fall, wird zusätzlich ein visueller und/oder akustischer Alarm durch das Display 44 und den Lautsprecher 46 abgegeben.

Bei dem in Figur 1 gezeigten Gerät hat das Netzteil 28 eine digitale Schnittstelle 281, die mit einem Ausgang des Prozessrechners 36 verbunden ist. Durch die Schnittstelle 28I ist die Spannung steuerbar, welche das Netzteil 28 angibt.

Damit kann der Prozessrechner 36 nach einem Erkennen eines Handstückes, welches mit einer kleineren Standardspannung erfolgt, für den Betrieb des Handstückes die vom Netzteil 28 abgegebene Spannung erhöhen, und zwar auf je nach Handstück unterschiedliche Werte, die ebenfalls in der Gerätetabelle des Speichers 42 abgelegt sind (4.oder weitere Spalte).

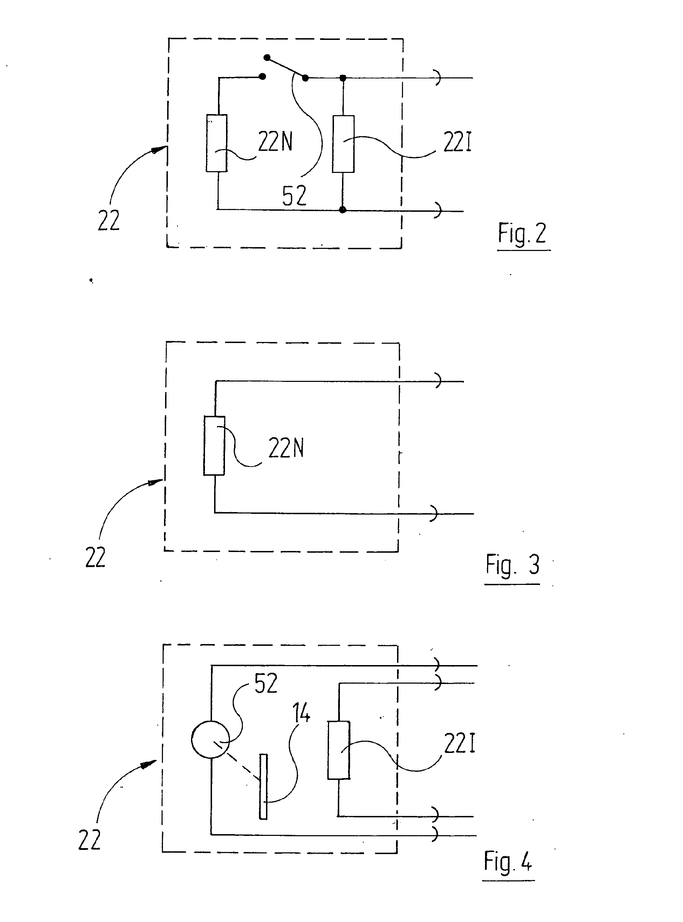

Wie aus Figur 2 ersichtlich, kann ein Verbraucher 22 typischerweise einen Nutzverbraucher 22N und einen Identifizierungsverbraucher 22I umfassen, die parallel zwischen die beiden Versorgungsleitungen geschaltet sind. Dabei kann der Betrieb des Nutzverbrauchers 22N durch Schließen und Öffnen eines Schalters 50 gesteuert werden.

Damit kann das Identifizieren des Handstückes 10 erfolgen, ohne dass der Nutzverbraucher 22N eingeschaltet zu werden bräuchte.

Figur 3 zeigt einen abgewandelten Verbraucher 22, der nur einen Nutzverbraucher 22N umfasst. Dieser ist ständig mit den Versorgungsleitern verbunden und kann somit die Aufgabe eines Identifizierungsverbrauchers mit übernehmen. Um zu verhindern, dass der Nutzverbraucher 22N schon dann arbeitet, wenn das Handstück 10 in das Basisteil 16 gesteckt wird, ist das Netzteil 28 so voreingestellt, dass es zunächst nur eine zum Betreiben des Nutzverbrauches 22N nicht ausreichende Spannung bereitstellt. Erst dann, wenn die Identifizierungsphase beendet ist (Ende des Ausgangssignales der Kippstufe 38) wird dann das Netzteil 28 vom Prozessrechner 36 auf die normale Arbeitsspannung eingestellt.

Figur 4 zeigt einen Verbraucher 22, der keinen elektrischen Verbraucher enthält, nur fluidische Verbraucher hat, die z.B. eine Druckluftturbine 52, welche einen Bohrer antreibt.

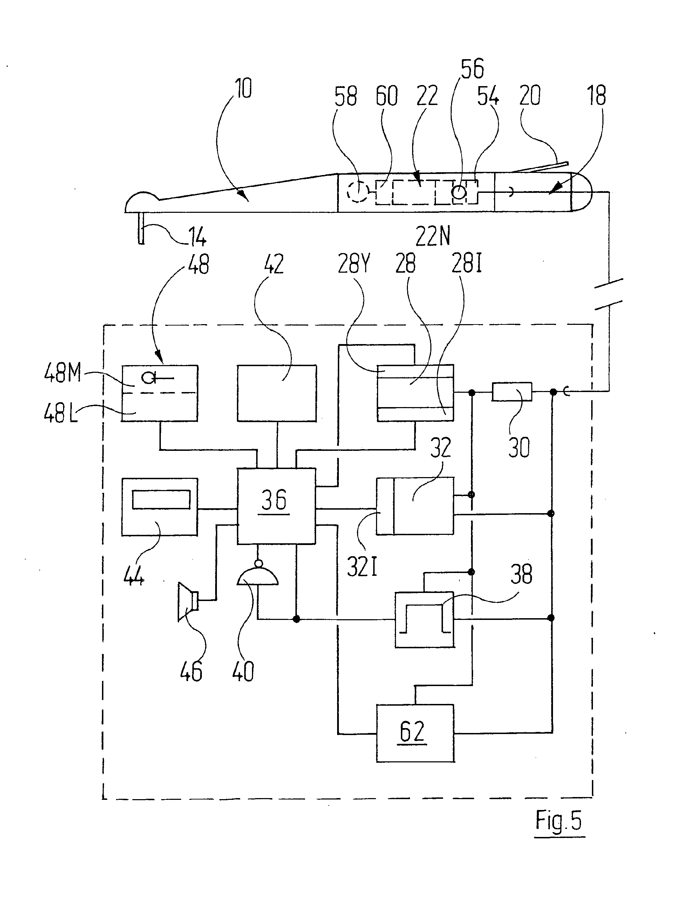

Beim Ausführungsbeispiel nach Figur 5 sind Teile des Arbeitsgerätes, die obenstehend unter Bezugnahme auf die Figur 1 schon beschrieben wurden, wieder mit denselben Bezugszeichen versehen. Sie brauchen nachstehend nicht nochmals erläutert zu werden.

Das Arbeitsgerät nach Figur 5 ist nun zusätzlich so ausgestaltet, dass digitale Daten von der Versorgungseinheit 12 zum Handstück 10 und umgekehrt vom Handstück 10 zur Versorgungseinheit 12 übermittelt werden können.

Diese Übermittlung erfolgt durch hochfrequente Modulation des Versorgungsstromes.

Das Übertragen von Signalen von der Versorgungseinheit 12 zum Handstück 10 erfolgt durch entsprechende Steuerung des Netzteiles 28 unter Verwendung des Interfaces 281. Die entsprechenden Modulationen sind verglichen mit der normalen Arbeitsspannung gering, so dass sie das Arbeiten des Handstückes 10 nicht nenenswert beeinflussen.

Das Handstück 10 hat seinerseits einen Demodulator 54, der einen Hochpass und einen Seriell/Parallel-Umsetzer umfasst. Der Ausgang des Demodulators 54 ist mit einem Aktor verbunden, der auf einen der Nutzverbraucher 22N einwirkt.

Im Inneren des Handstückes 10 ist ein Sensor 58 angeordnet, der auf einen der aktuellen Arbeitsparameter des Handstückes 10 anspricht, z.B. auf die Temperatur eines Motors, welcher den Bohrer 14 bewegt. Das Ausgangssignal des Sensors 58 wird in einem Modulator 60 dazu verwendet, den durch das Handstück 10 fließenden Strom hochfrequent zu modulieren, wobei die Amplitude der so erzeugten hochfrequenten Anteile wieder nur einen kleinen Bruchteil der Amplitude des Versorgungsstromes darstellt, so dass das Arbeiten des Handstückes 10 wieder nicht nennenswert beeinflusst wird.

Der so mudulierte Versorgungsstrom fließt über den Wierstand 30 und an dessen Klemmen ist ein Demodulator 62 angeschlossen, der wiederum ein Hochpassfilter und einen nachgeschalteten Seriell/Parallel-Umsetzer umfasst. Das Ausgangssignal des Demodulators 62 wird auf einen weiteren Eingang des Prozessrechners 36 gegeben, der in Abhängigkeit vom so übertragenen Ist-Arbeitsparameter des Handstückes 10 die Versorgungsspannung ändern kann (nach Amplitude und/oder Frequenz).

Dem Netzteil 28 ist ein digitaler Spannungsmesser 28V zugeordnet, welcher die vom Netzteil abgegebene Ist-Spannung misst (bezüglich Amplitude und ggfs. auch Frequenz).

Man erkennt, dass ein Arbeitsgerät gemäß Figur 5 Fehler im Arbeiten des Handstückes 10 rasch dem Benutzer mitteilen kann, und bezüglich seines Arbeitens von der Versorgungseinheit 12 her gesteuert werden kann.

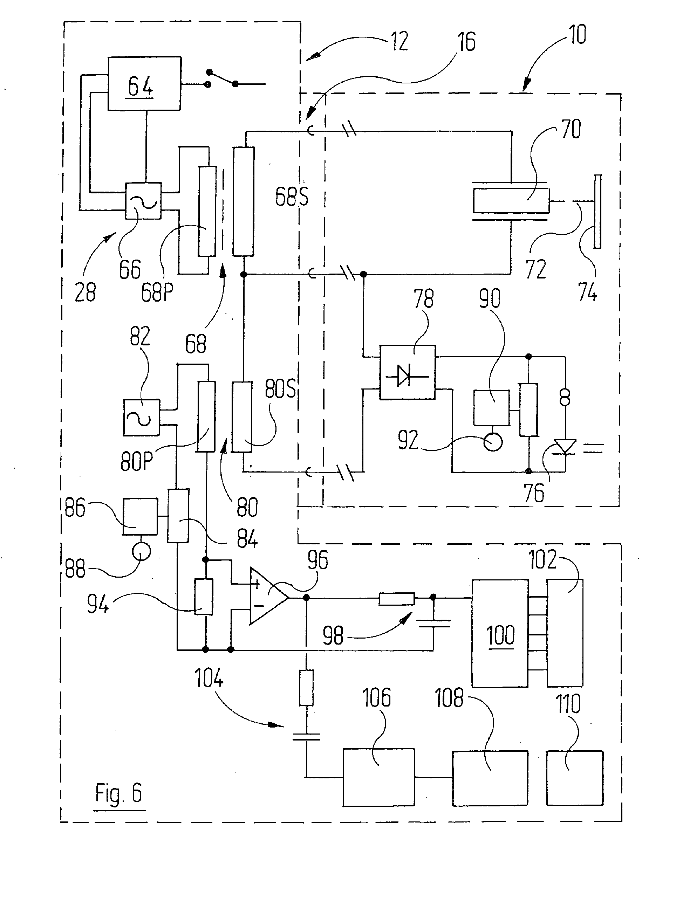

Figur 6 zeigt ein Ausführungsbeispiel eines dentalen Ultrasschallgerätes, bei welchem die oben beschriebenen Funktionen der Erkennung des Handstückes unter Verwendung einfacher handelsüblicher Standard-Komponenten und Standard-Schaltkreise erfolgt.

Das Netzteil 28 umfasst ein Steuergerät 64, welches eine Gleichspannung abgibt, die von einem Wechselrichter 66 in eine Wechselspannung umgesetzt wird. Die Steuerung des Wechselrichters 66 erfolgt ebenfalls durch das Steuergerät 64, so dass am Ausgang des Wechselrichters 66 eine Wechselspannung einstellbarer Frequenz und Amplitude erzeugt wird.

Der Wechselrichter 66 ist mit der Primärspule 68P eines Betriebsspannungs-Transformators 68 verbunden. Dessen Sekundärspule 68S ist mit den Arbeitsklemmen eines Schwingquarzes 70 verbunden. Dieser arbeitet über eine nur schematisch dargestellte mechanische Koppelstrecke 72 auf ein Ultraschallwerkzeug 74.

Ein weiterer Verbraucher des Ulstraschall-Handstückes 10 ist eine Leuchtdiode 76, welche die Arbeitsstelle des Ultraschallwerkzeuges 74 beleuchtet. Die Leuchtdiode 76 wird durch einen Gleichrichter 78 gespeist, der mit der Sekundärwicklung 80P eines Niederspannungs-Transformators 80 verbunden ist.

Dessen Primärwicklung 80P wird von einer Wechselspannungsquelle 82 versorgt. In den Primärstromkreis ist ein steuerbarer Widerstand 84 geschaltet, der durch einen Treiberkreis 86 gesteuert wird. Dieser setzt das Ausgangssignal eines Befehlsgebers 88 in eine Modulationsamplitude/ oder eine Modulationsphase um, welche dem Grundstrom durch die Primärspule 80P aufgeprägt wird.

Der Widerstand 84 kann Ohm'sche und/oder kapazitive und/oder induktive Komponenten enthaöten, von denen mindestens eine steuerbar ist.

Ein Treiberkreis 90, der in Abhängigkeit eines Sensors 92 arbeitet, der auf einen Arbeitsparameter des Handstückes anspricht, ist über die Ausgangsklemmen des Gleichrichters 78 geschaltet. Auf diese Weise kann der vom Gleichrichter zu liefernde Strom gemäß dem zu überwachende Ist-Parameters des Handstückes ausgehend von einem Mittelwert erhöht oder erniedrigt werden.

Der Treiberkreis 86 und der steuerbare Widerstand 88 bilden zusammen eine Quelle für vom Handstück auf die Versorgungseinheit zu übertragende Signale.

Die beiden Eingangsklemmen des Gleichrichters 78 sind mit der einen Versorgungsleitung für den Schwingquarz 70 verbunden, die andere Eingangsklemme des Gleichrichters 78 ist mit der einen Klemme der Sekundärwicklung 68F des Transformators 68 verbunden. Die andere Klemme der Sekundärspule 68S ist mit der einen Versorgungsleitung des Schwingquarzes 70 verbunden, wie aus der Zeichnung ersichtlich.

In den Primär-Stromkreis des Transformators 68 ist ferner ein Widerstand 94 zum Messen des im Primärkreis fließenden Stromes geschaltet. Dessen beide Klemmen sind mit den Eingangsklemmen eines Differenzverstärkers 96 verbunden, der über einen RC-Tiefpass 98 mit dem Eingang eines grob gerastert arbeitenden digitalen Spannungsmessers 100 verbunden ist. Durch dessen Ausgangssignal wird eine Anzeige 102 gesteuert.

Über einen RC-Hochpass 104 werden hochfrequente Anteile des Versorgungsstromes auf einen impulsformenden Seriell/- Parallelwandler 106 gegeben, dessen Ausgang mit einem Dekodierer 108 und einer Anzeige 110 verbunden ist.

Wird bei dem in Figur 6 gezeigten Arbeitsgerät das Handstück 10 mit der Versorgungseinheit 12 verbunden, so liegt am Ausgang des Gleichrichters 78 Spannung. Die Leuchtdiode 76 leuchtet nun und die Sekundärspule 68S des Transformators 68 muss eine entsprechende Leistung bereitstellen. Ein entsprechender Speisestrom fließt dann im Primärkreis des Transformators 68, und dieser Strom wird über den Differenzverstärker 96, den Tiefpass 98 und den Spannungsmesser 100 gemessen.

Dieser arbeitet so, dass er z.B. 32 Spannungssignale unterschiedlicher Größe auflösen kann. Das entsprechende Ausgangssignal des Spannungsmessers 100 kann direkt in eine entsprechende digitale Anzeige auf dem Display 102 umgesetzt werden.

In Abhängigkeit vom Arbeitsparameter des Handstückes, auf welchen der Sensor 92 anspricht, wird dem Strom im Primärkreis des Transformators 68 eine hochfrequente Komponente aufgeprägt. Diese gelangt über den Hochpass 104 auf den Decoder 106 und führt zu einer entsprechenden Darstellung auf dem Display 110. Auf diese Weise kann das Arbeiten des Handstückes einfach überwacht werden.

Die Stufen der Strommessung, die bei den oben beschriebenen Arbeitsgeräten verwendet werden, entsprechen Sollstrombereichen, in denen ein Arbeitskopf identifiziert wird.

Obenstehend wurde ein Arbeitsgerät mit der Erkennung eines Arbeitskopfes und Beeinflussbarkeit des Arbeitens anhand eines elektrischen Arbeitsgerätes mit elektrischen Verbrauchern und unter Verwendung elektrischer Signale beschrieben. Es versteht sich, dass ein solches Arbeitsgerät dann mit elektrischem Strom auch mit einem Fluidstrom und statt elektrischer Signale auch mit Fluidsignalen arbeiten kann.

## Patentansprüche

1. Arbeitsgerät mit einem wechselbaren Arbeitskopf (10), insbesondere einem dentalen Handstück, mit einem mit dem Arbeitskopf (10) lösbar verbindbaren Basisteil (18) und mit einer mit dem Basisteil (18) verbundenen Versorgungseinheit (12), welche einen Versorgungsstrom für den Arbeitkopf (10) bereitstellt, **dadurch gekennzeichnet, dass** die Versorgungseinheit (12) einen Strommesser (30, 32), welcher den Versorgungsstrom misst, und eine mit dem Strommesser (30, 32) verbundene Auswertelogik (36, 42) umfasst, welche das gemessene Iststromsignal nach Größe und/oder Phasenlage misst und mit mindestens einem Sollstrombereich vergleicht und als Ergebnis dieses Vergleichs ein Handstückidentifizierungssignal bereitstellt.

2. Arbeitsgerät nach Anspruch 1, **dadurch gekennzeichnet, dass** die Auswertelogik (36, 42) das Iststromsignal mit einer Vielzahl von Sollstrombereichen vergleicht und ein Ausgangssignal bereitstellt, welches eindeutig mit demjenigen Sollstromfenster korreliert ist, in welches das Iststromsignal fällt.

3. Arbeitsgerät nach Anspruch 1 oder 2, **dadurch gekennzeichnet, dass** durch einen Stromfühler (30) des Strommessers (30, 32) ein monostabiler Taktgeber (38) angestoßen wird, welcher das Arbeiten der Auswertelogik (36, 42) steuert.

4. Arbeitsterät nach einem der Ansprüche 1 bis 3, **dadurch gekennzeichnet, dass** die Auswertelogik (36, 42) mit einer Sollidentifizierungssignalquelle (48) verbunden ist und ein Alarmsignal erzeugt, wenn das Sollidentifizierungssignal nicht mit dem ermittelten Istidentifizierungssignal übereinstimmt.

5. Arbeitsgerät nach Anspruch 4, **dadurch gekennzeichnet, dass** die Sollidentifizierungssignalquelle (48) ein Mikrofon (48M) und eine dieser nachgeschaltete Spracherkennungslogik (48L) umfasst.

6. Arbeitsgerät nach einem der Ansprüche 1 bis 5,
**dadurch gekennzeichnet, dass** die Auswertelogik (36, 42) nach Ablauf einer vorgegebenen Zeitspanne (38) die mit dem Verbinden von Arbeitskopf (10) und Versorgungseinheit (12) oder mit dem Einschalten eines im Arbeitskopf (10) befindlichen Verbrauchers (22N) beginnt, in einen Überwachungsmodus übergeht,
in welchem das Iststromsignal mit einem Soll-Betriebsstromsignal vergleichen wird.

7. Arbeitsgerät nach einem der Ansprüche 1 bis 6,
**dadurch gekennzeichnet, dass** der Arbeitskopf (10) einen Modulator (60) für den Versorgungsstrom aufweist, der durch einen auf einen Arbeitsparameter des Arbeitskopfes (10) ansprechenden Sensor (58) gesteuert wird, und die Auswertelogik (36, 42) mit einem passenden Demodulator (62) zusammenarbeitet, der in den Stromkreis des modulierten Versorgungsstromes geschaltet ist und dem gemessenen Betriebsparameter wiedergewinnt.

8. Arbeitsgerät nach einem der Ansprüche 1 bis 7,
**dadurch gekennzeichnet, dass** die Versorgungseinheit (12) einen Modulator (28I; 84) aufweist, der durch eine Steuersignalquelle (88; 36) gesteuert wird und der Arbeitskopf (10) einen passenden Demodulator (54) aufweist, der aus dem modulierten Versorgungsstrom das Steuersignal wieder gewinnt.

9. Arbeitsgerät nach Anspruch 7 und Anspruch 8, **dadurch gekennzeichnet, dass** die Modulationsfrequenzen für die Übertragung des Ist-Arbeitsparameters und für die Übertragung des Steuersignals unterschiedlich sind.

10. Arbeitsgerät nach einem der Ansprüche 1 bis 9, **dadurch gekennzeichnet, dass** der Arbeitskopf (10) einen Identifizierungswiderstand (221) aufweist, der von zumindest einem Teil des Versorgungsstromes durchflossen wird, wenn der Arbeitskopf (10) mit der Versorgungseinheit (12) verbunden wird.

11. Arbeitgerät nach einem der Ansprüch 1 bis 10, **dadurch gekennzeichnet, dass** der Arbeitskopf (10) einen Nutzverbraucher (22N) und einen Identifizierungswiderstand (22I) aufweist, und dieser Verbraucher (22N) und der Identifizierungswiderstand (22I) einen Versorgungsleiter gemeinsam haben.

12. Arbeitsgerät nach Anspruch 10 oder 11, **dadurch gekennzeichnet, dass** die Stromversorgung des Indentifizierungsverbrauchers (22I) eine vorgegebene Zeitspanne nach ihrem Beginn oder nach Erkennen des Arbeitskopfes (10) beendet wird.
